# EUROPEAN PATENT APPLICATION

(11) **EP 1 502 625 A1**
(43) Date of publication of application: **02.02.2005**
(21) Application number: 04017621.6
(22) Date of filing: 26.07.2004
(51) Int. Cl.: A61N 5/10

(54) **A system for processing patient radiation treatment data**

(30) Priority: 30.07.2003 US 491211 P; 07.10.2003 US 680347
(71) Applicant: Siemens Medical Solutions Health Services Corporation, Malvern, PA 19355 (US)
(72) Inventor: Fitzgerald, Loretta A., 19426 Collegeville PA (US)
(74) Representative: Payne, Janice Julia

(57) **Abstract**

A system records and documents both external beam radiation therapy and radiation from implanted sources applied to an anatomical area, in a comprehensive consolidated record of radiation treatment that also documents detected radiation emission levels from internal patient sources measured externally to the patient. A system processes data concerning patient radiation treatment. The system includes an input processor for receiving data identifying a first radiation dose received by a particular anatomical part of a patient, from a radiation source external to a patient and a second radiation dose, received by the particular anatomical part of the patient from a radiation source internal to the patient. A data processor combines data representing the first and second dose to provide a combined dose value. A storage processor stores the combined value in a record associated with the patient.

## Description

This invention concerns a system for processing radiation treatment data from multiple different sources.

Existing treatment planning systems are used to determine a radiation dose distribution occurring within a patient as a result of radiation beam therapy external to the patient or from implanted radiation sources (brachytherapy). Such existing systems include electronic systems which document the radiation treatments a patient receives from external beam radiation therapy and which provide a means of showing a daily given dose and a cumulative dose. Also existing treatment planning systems are used to generate a treatment plan for brachytherapy as documentation for addition to a patient treatment record. A radiation oncologist typically dictates a treatment note, documenting a radiation dose a patient received at an anatomical treatment site and surrounding anatomical structures. However, existing treatment planning systems fail to process data received from both external beam radiation therapy and brachytherapy in a manner that facilitates radiation treatment planning and provides a user friendly comprehensive record of radiation treatment suitable for incorporation in an electronic patient record and presentation to a clinician. A system according to invention principles addresses these deficiencies and associated problems.

A system records and documents both external beam radiation therapy and radiation from implanted sources in a manner that facilitates patient radiation treatment planning and provides a user friendly comprehensive consolidated record of radiation treatment by both therapies in one place in an electronic patient record. A system processes data concerning patient radiation treatment. The system includes an input processor for receiving data identifying a first radiation dose received by a particular anatomical part of a patient, from a radiation source external to a patient and a second radiation dose, received by the particular anatomical part of the patient from a radiation source internal to the patient. A data processor combines data representing the first and second dose to provide a combined dose value. A storage processor stores the combined value in a record associated with the patient.

An example of the present invention will now be described with reference to the accompanying drawings in which:-
Figure 1 shows functions of a healthcare enterprise incorporating a radiation data processing and treatment planning system, according to invention principles.
Figure 2 shows a flowchart of a workflow process employed in a radiation treatment and data processing system, according to invention principles.
Figure 3 shows a radiation implant record, according to invention principles.
Figure 4 illustrates a type of radiation implant source device, according to invention principles.
Figure 5 illustrates a survey record for documenting radiation strength from radiation sources internal to a patient, according to invention principles.
Figure 6 illustrates a radiation therapy and treatment planning record for inclusion in a patient record for documenting external radiation beam therapy and internal implant radiation therapy, according to invention principles.
Figure 7 depicts room locations corresponding to some of the measurement locations (locations 1-6) detailed in the survey record of Figure 5, according to invention principles.

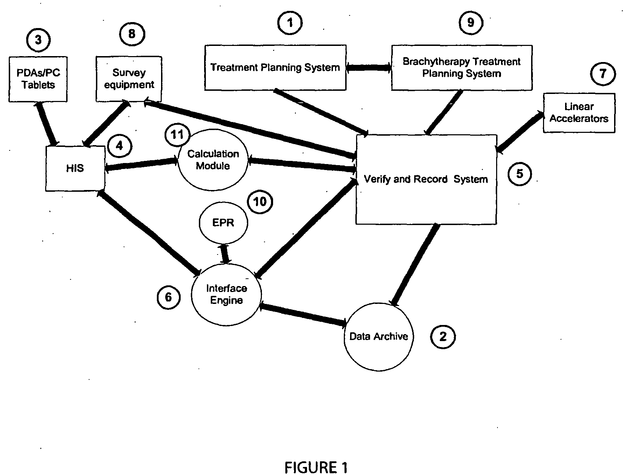

The inventor has advantageously recognized the need for an electronic system for processing and adding patient external radiation dose and implant radiation dose information to provide data identifying a cumulative radiation dose applied to a patient anatomical area. Figure 1 shows functions of a healthcare enterprise incorporating a radiation data processing and treatment planning system. The radiation data processing and treatment planning system provides processed radiation data in a user friendly display image presentation. The system also provides the processed radiation data as a consolidated treatment record for storage in an electronic patient record readily used by a clinician or another electronic system to improve healthcare delivery effectiveness and efficiency. During the course of treating patients with cancer, for example, increasingly, patients are treated with multi-modalities such as surgery, chemotherapy, and radiation therapy. Patients may receive radiation therapy from external beam radiation sources or implanted sources (Brachytherapy). Presently, systems exist to document external beam treatments but these systems fail to document radiation delivered from implanted sources or to process and record consolidated radiation data from both types of sources.

In order to comply with Nuclear Regulatory Commission (NRC) regulations, radiation measurement procedures are followed to survey and document radiation levels external to a patient resulting from radiation sources implanted within the patient. Upon completion of an implant treatment process and removal of the implanted radioactive material, the patient, room, trash, and patient clothing is surveyed to measure and document that the patient and the items that have come in contact with the patient, are substantially free of radiation. In the case of a permanent radioactive implant, in which the radioactive sources remain in the patient, a survey of a patient is performed and documented. This is to ensure that radiation measurements are within the NRC guidelines and that it is safe for an implanted patient to be free to move within the public and that the patient does not pose a risk to the general public or immediate family.

In the Figure 1 system, treatment planning system 1 supports user planning of external beam radiation therapy and treatment planning system 9 supports planning of radiation therapy using an implanted source. The implanted radiation source therapy includes High Dose Rate (HDR) therapy (which may include an applicator for encapsulating a radiation source), Low Dose Rate therapy as well as Temporary and Permanent implants. In the treatment of coronary artery disease, for example, 10 to 20 percent of patients develop restenosis following angioplasty or in-stent restenosis following a stent procedure. Intravascular implant radiation comprising implanting a small amount of radiation at a reopened intravascular area is used to inhibit renarrowing. Treatment planning systems generate isodose plans, which are a visual representation of the radiation dose distribution within a patient and may indicate doses as percentages of a maximum value.

Treatment planning data derived using planning system 1 is used by Verify and Record (V&R) system 5 to validate that configuration settings of linear accelerator 7 are appropriate for delivering a desired external beam radiation therapy dose to a patient. Specifically, Verify and record system 5 processes treatment planning data from system 1 to create and store parameters for each treatment field for linear accelerator 7. During treatment of a patient, a field is selected and V&R system 5 automatically sets required field parameters of linear accelerator 7 via an interface to accelerator 7. In response to user initiation of treatment, V&R system 5 verifies that accelerator 7 parameters which are physically set on accelerator 7 are within ranges defined in an associated treatment plan provided by system 1.

Once treatment has been given, data identifying the treatment delivered is stored by system 5 in electronic patient record 10 via a communication network and interface engine 6. The patient radiation therapy treatment record, including daily radiation therapy treatment records created by verify and record system 5, is stored and maintained in EPR 10 or archive 2, but may also be stored and maintained elsewhere. Verify and record system 5 may also display an external beam treatment record and an implant radiation treatment record and may display cumulative radiation dose data. The data identifying the treatment delivered that is stored in EPR 10 includes, accelerator 7 parameters, treatment time, tumor radiation dose, total cumulative radiation dose (including dose provided from implanted sources), duration of treatment and treatment area.

Data representing treatment plans derived from systems 1 and 9 are stored in data repository 2 and EPR 10 by V&R system 5 and are accessible in response to a user access request. Treatment plans (external beam and implant radiation) and related data are transmitted via standard communication protocols and data formats including DICOM PS 3.3-2003 A30.3 (or by other e.g., non-standard means). Treatment plans and radiation treatment data stored in EPR 10 or archive 2 are accessible by a clinician using a portable Personal Data Assistant (or laptop, etc.) 3 supporting remote data entry and access capability by communicating through an interface with Hospital Information System (HIS) 4. Portable devices 3 will provide wireless access to HIS 4 enabling a healthcare worker to enter patient related data from a remote location. Further, HIS 4 may incorporate EPR 10 and other treatment records. In addition, HIS 4 (or V&R system 5) incorporates a rules processor for generating alert messages identifying excessive or inappropriate radiation dose settings and exposure A workflow processor within HIS 4 schedules, automates and manages a sequence of tasks involved in producing treatment plans in conjunction with systems 1 and 9 and in scheduling personnel and related work task sequences to optimize the delivery of healthcare to a patient. HIS 4 also receives radiation survey data from Radiation measurement equipment 8 (such as an ion chamber) and processes the data for storage in EPR 10 or repository 2 via interface 6.

In an exemplary calculation, Implant therapy planning system 9 automatically sums a patient external radiation dose and an implant radiation dose to provide data identifying a cumulative radiation dose applied to a patient anatomical area. System 9 determines that a patient receiving 4500cGy radiation to an area from external beam therapy and an additional dose of 2000cGy to the area from an implanted radiation source receives a total dose of 6500cGy to this area, for example. System 9 automatically updates EPR 10 via system 5 and interface 6 to record the total dose information for the patient. Doses to identified dose points from systems 9 and 1 are added together to determine the cumulative radiation dose. In another embodiment of this invention, the calculation may be performed in system 1, 5, 4, or a separate calculation module (system 11). HIS 4 and V&R System 5 also electronically store patient radiation survey records derived from Radiation measurement equipment 8, as well as other radiation exposure records, non-medical treatment associated sources, and radiation received by personnel caring for the patient, in EPR 10 via interface 6. Radiation survey equipment that is interfaced will import data into HIS 4 or V&R System 5 directly. When no interface is enabled, and if personnel caring for the patient document their exposure time on paper, the HIS 4 enables a user of system 8 to employ a predetermined template form, or to modify a predetermined template form to record radiation readings obtained at specific distances from a patient implanted with a radioactive material for any brachytherapy procedure.

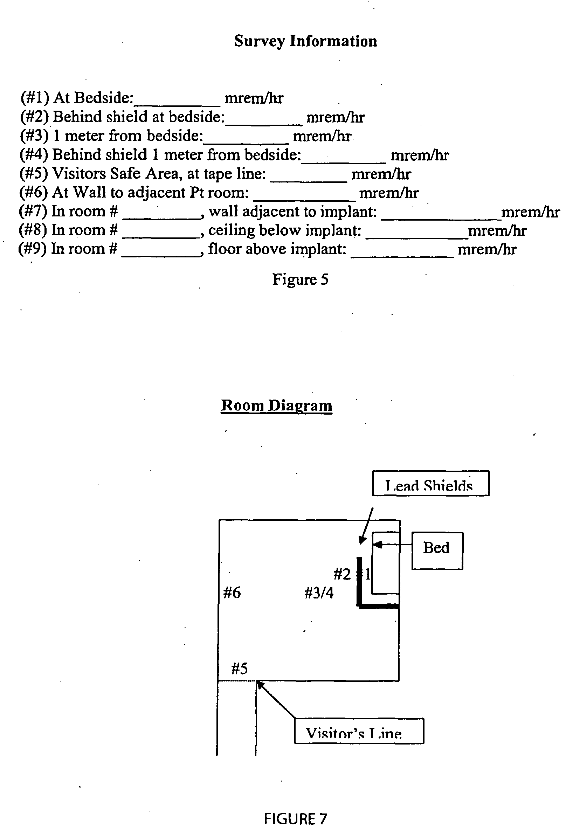

Figure 5 illustrates a radiation survey template form for documenting radiation strength from radiation sources internal to a patient. Template survey forms vary depending upon the implant radiation procedure performed and radiation source used. Figure 5 items 1-9 identify different locations at which patient radiation measurements are made using equipment 8. Items 1-6 indicate locations within a patient room that are diagrammatically depicted in Figure 7. Further, Figure 7 also illustrates a visitor line nominally indicating a safe area threshold and also shows lead shielding used to contain patient radiation emission within the patient room. The Figure 5 template form is readily adaptable to capture appropriate readings for a selected type of implant radiation source. HIS 4 electronically stores patient radiation measurements made using Radiation measurement equipment 8 recorded in the template form of Figure 5. The measurements are stored in EPR 10 or archive 2 via interface 6. The system of Figure 1 illustrates communication paths between Radiation measurement equipment 8 and the rest of the system. These communication paths may be implemented using wired or wireless communication. Further, PC or PDA 3 are used for displaying a Figure 5 template form to a user to support manual data entry. Alternatively in another embodiment measurement data from equipment 8 is automatically stored in designated data fields in the form.

The template form illustrates exemplary data fields for receiving radiation measurements made external to a patient that has received a temporary implant during a hospitalization period. Additional data fields are described below. The radiation measurements are performed shortly after a patient has been implanted with a radionuclide source. The radiation is measured in mrem/hr at locations external to the patient including, at the bedside, one meter from bedside, in a visitor safe area, at a doorway to the patient room and in the surrounding areas, for example. In addition to the radiation measurement rates, the template form record includes items (not shown in Figure 5) comprising, patient name, patient location, date and time of measurement, Isotope Number of source, source strength and total source strength if multiple sources are involved. A completed template form is stored within a designated section of the patient EPR 10 or data is extracted from the form and stored in a desired format in EPR 10. Input of survey data may be manual either from PC keyboard or via wireless technology.

The wireless access to HIS 4 provided by portable device 3 facilitates healthcare worker entry of patient radiation survey data for storage in EPR 10. This documentation is stored with the patient record to meet NRC requirements. The system advantageously stores different radiation records together in EPR 10, an electronic medium and not in a commonly used paper chart. Paper charts currently in widespread use for the purpose of recording radiation therapy data, are frequently misplaced, corrupted, damaged or misfiled. In contrast, a complete electronic patient record (such as EPR 10) incorporating comprehensive radiation data as previously described (including total dose and other radiation data) in a consolidated manner, facilitates efficient, user friendly access to patient radiation data. EPR 10 also advantageously contains data for use in demonstrating compliance with regulations.

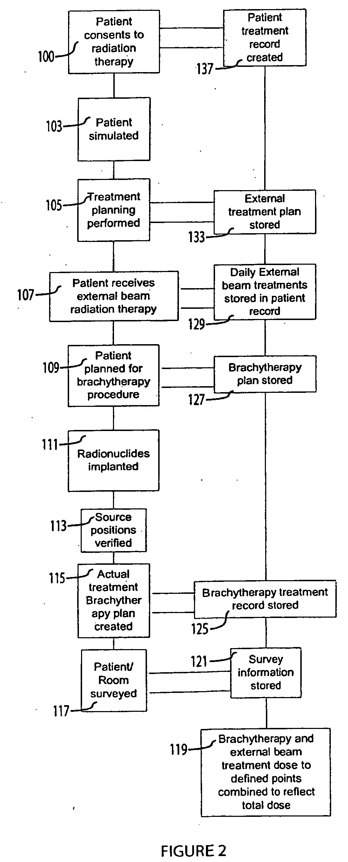

Figure 2 shows a flowchart of a workflow process employed in the radiation treatment and data processing system of Figure 1. Following patient consent to radiation therapy in step 100, a patient radiation treatment record is generated in step 137 and a simulation of the patient is conducted in step 103 for use in treatment planning in step 105. The simulation involves constructing a three dimensional model of the target patient anatomical area requiring radiation treatment for use in determining optimal dosage and disposition of a radiation beam with respect to the target area. Treatment planning performed in step 105 determines the radiation dosage, number and timing of external radiation beam treatments and location and disposition of the beam with respect to the target anatomical area. The produced treatment plan is stored in the generated radiation treatment record in step 133. The external beam radiation treatment is applied to the patient in step 107 and data identifying the applied individual treatments is stored in the generated radiation treatment record in step 129.

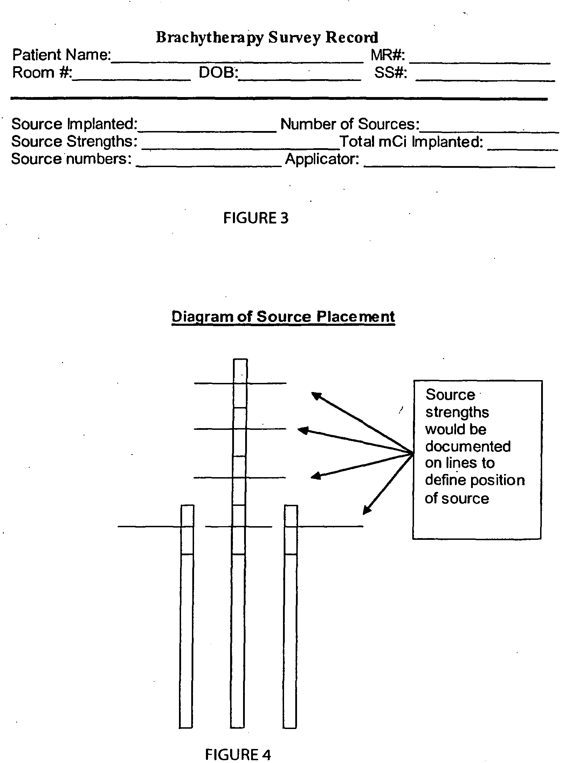

In step 109, radiation therapy based on an implanted source is planned for the patient and the resulting treatment plan is stored in the generated radiation treatment record in step 127. A radionuclide radiation source is implanted into the patient at the target anatomical area in step 111 and the position of the sources is verified both during implanting and by subsequent imaging in step 113. A second radiation treatment plan (reflecting the treatment actually applied) is generated in step 115 based on the strength, type and position of the actual implanted source. The treatment plan is stored in the generated radiation treatment record in step 125. Figure 3 illustrates a radiation implant record for documenting a radiation implant treatment and characteristics of an implanted source. The implant treatment record incorporates data fields appropriate to a particular type of implant (e.g., radiation source encapsulation rods, seeds etc.). An implant treatment record includes as shown in Figure 3, Patient name, Medical Record Number, Social Security Number, Date of Birth, Room number, identification of source implanted, number of sources, source strength, total mCi implanted, source numbers and applicator, for example. An implant treatment record may also include, Date/Time, implant Technique (e.g. intravascular, intracavular, interstitial, intralumenary), implant Type (e.g., Manual, LDR - Low Dose rate, HDR - High Dose Rate, PDR - Pulsed Dose Rate, MDR - Medium Dose Rate), Treatment Site, Isotope, Date/time inserted, Date/time removed, Treatment time, Dose Rate, Isodose, Description of treatment site and Dose Delivered and Description of both critical structures and Dose Delivered. Further, the implant treatment record may be compatible with a DICOM A.30.3 RT Brachy Treatment Record IOD Module Table, for example. An implant radiation treatment record is used to document the radiation a patient received from implant radiation to a tumor area and additional points of interest.

Figure 4 illustrates a type of radiation implant device for encapsulating radiation sources. Specifically, Figure 4 shows a Fletcher-Suite Applicator. This applicator is an intra-cavitary device which is used to treat gynecologic cancer, for example. The applicator consists of three tandems (containment tubes), which comprise hollow steel tubes. Cesium sources are loaded into plastic tandems which are inserted into the applicator tubes. A center tandem may contain 3-5 sources, for example, depending upon a Radiation Oncologist prescription. In addition, the individual Cesium sources may have different strengths. The position of the sources and their various strengths determine a radiation dosage output of the treatment plan and is documented in the treatment record of Figure 3.

The patient is surveyed in step 117 to determine radiation emissions from the patient implanted source at fixed distances from the patient. This is to verify that contact by other persons with the patient is safe and that it is safe to let the patient leave the healthcare facility. Similarly, patient clothing and other items in the patient room are measured to validate they are safe. The resulting measurements are stored in the generated radiation treatment record in step 121 and are available to verify the treatment complies with NRC (Nuclear Radiation Commission) guidelines. The dose received by the patient target anatomical area from the external radiation beam source and implanted source are added to provide a combined (total) dose value for storage in step 119 in the consolidated generated radiation treatment record providing a single point for review of patient progress during radiation therapy.

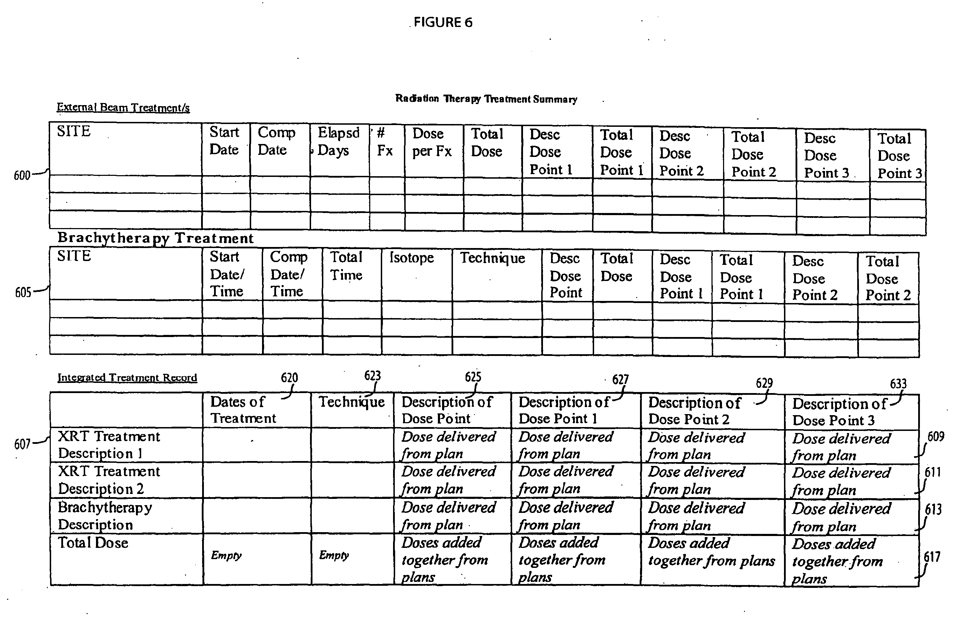

Figure 6 illustrates a consolidated radiation therapy and treatment planning record for inclusion in electronic patient record (EPR 10 of Figure 1). The record documents external radiation beam therapy and internal implant radiation therapy plans. Planned external radiation treatment are documented in record section 600, planned implant radiation treatments are documented in record section 605 and a planned integrated radiation treatment record documenting both external and implant radiation treatments and combined dosage values is shown in record section 607. In integrated record section 607, planned external radiation treatments for multiple anatomical sites are documented in rows 609 and 611 and planned implant radiation treatments for the multiple anatomical sites are documented in row 613. Row 617 documents combined planned external radiation treatment and implant radiation treatment values for the multiple anatomical sites. Columns 620-633 of integrated record section 607 show planned individual radiation treatment values for individual treated anatomical sites together with associated treatment dates and descriptions of the treatment technique applied. Specifically, column 620 documents planned treatment dates, column 623 documents planned treatment technique and columns 625 - 630 document the planned treatment values for individual treated anatomical sites. Thereby the system provides documentation of an implant radiation dose in an electronic record and a means of consolidating external beam and implant radiation doses in a patient treatment record (e.g., EPR 10 Figure 1). The stored EPR 10 data is readily accessible both electronically and in printed form and the total dose value reflects the combined radiation dose administered through external beam and implant radiation techniques.

The system, processes and record configurations presented in Figures 1-6 are not exclusive. Other system, processes and record configurations may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention.

## Claims

1. A system for processing data concerning patient radiation treatment, comprising:
an input processor for receiving data identifying a first radiation dose, received by a particular anatomical part of a patient, from a radiation source external to a patient and a second radiation dose, received by said particular anatomical part of said patient from a radiation source internal to said patient;
a data processor for combining data representing said first and second dose to provide a combined dose value; and
a storage processor for storing said combined value in a record associated with said patient.

2. A system according to claim 1, wherein
said radiation source internal to said patient is a device implanted within said patient and
said data processor combines said data representing said first and second dose by summing values representing said first and second dose to provide said combined dose value.

3. A system according to claim 1, wherein
said radiation source internal to said patient comprises at least one of, (a) an implanted device, (b) an intra-vascular radiation treatment source, (d) a temporarily inserted radiation source, (e) a permanently inserted radiation source, (f) a diagnostic related radiation source and (g) a therapeutic radiation source.

4. A system according to any preceding claim, wherein
said storage processor stores said combined value in an electronic patient record associated with said patient in a repository accessible from remote devices.

5. A system according to any preceding claim, wherein
said input processor receives data identifying a radiation value emitted by a radiation source implanted within a patient and
said storage processor stores said emitted radiation value and said combined value in said record associated with said patient in at least one of, (a) the same portion of said record and (b) a related record section of said record, to consolidate radiation related data in a particular patient record section.

6. A system according to any preceding claim, wherein
said record associated with said patient includes at least one of, (a) an identification of said particular anatomical part of said patient, (b) said first radiation dose, (c) said second radiation dose and (d) data identifying a radiation value emitted by a radiation source implanted within a patient.

7. A system according to any preceding claim, including
a communication interface for initiating communication of an alert message to a user identifying said combined value associated with said patient.

8. A system according to any preceding claim, including
a task manager for updating a record indicating a task to be performed by including an additional task in response to said combined value and wherein
said additional task is to be performed by at least one healthcare worker and comprises a prompt to a healthcare worker to perform an action.

9. A system according to any preceding claim, including
an interface processor for using said combined value in configuring a radiation therapy device for delivering a radiation treatment to a patient.

10. A system for processing data concerning patient radiation treatment, comprising:
an input processor for receiving data identifying a first radiation dose, received by a particular anatomical part of a patient, from a radiation source external to a patient and a second radiation dose, received by said particular anatomical part of said patient from a radiation source internal to said patient;
a data processor for combining data representing said first and second dose to provide a combined dose value; and
a task manager for updating a record indicating a task to be performed by including an additional task in response to said combined value.

11. A system according to claim 10, wherein
said input processor receives data identifying a radiation value emitted by a radiation source implanted within a patient and
said task manager updates a record to indicate a perimeter is to be designated identifying an area around said patient to be avoided by other personnel.

12. A method for processing data concerning patient radiation treatment, comprising the activities of:
receiving data identifying a first radiation dose, received by a particular anatomical part of a patient, from a radiation source external to a patient and a second radiation dose, received by said particular anatomical part of said patient from a radiation source internal to said patient;
combining data representing said first and second dose to provide a combined dose value; and
storing said combined value in a record associated with said patient.
